# EUROPEAN PATENT SPECIFICATION

(11) **EP 2 684 862 B1**
(45) Date of publication and mention of the grant of the patent: **14.11.2018**
(21) Application number: 12726714.4
(22) Date of filing: 01.04.2012
(51) Int. Cl.: C07C 211/14, C07C 209/48

(54) **METHOD FOR PREPARING N-(2-AMINOETHYL)ETHANE-1,2-DIAMINE**
VERFAHREN ZUR HERSTELLUNG VON N-(2-AMINOETHYL-)ETHAN-1,2-DIAMIN
PROCÉDÉ DE PRÉPARATION DE N-(2-AMINOÉTHYL)ÉTHANE-1,2-DIAMINE

(43) Date of publication of application: 15.01.2014
(73) Proprietor: Wanhua Chemical Group Co., Ltd., Yantai, Shandong 264000 (CN)
(72) Inventor: LI, Fuguo, Yantai Shandong 264002 (CN); LI, Yuan, Yantai Shandong 264002 (CN); DING, Ke, Yantai Shandong 264002 (CN); ZHAO, Wenjuan, Yantai Shandong 264002 (CN); YU, Xueli, Yantai Shandong 264002 (CN); HUA, Weiqi, Yantai Shandong 264002 (CN)
(74) Representative: Kador & Partner PartG mbB
(86) International application number: PCT/CN2012/073459
(87) International publication number: WO 2013/149373

(56) References cited:
- WO-A1-2008/104552
- WO-A2-2004/066703
- CN-A- 101 622 222
- CN-A- 101 622 223
- CN-A- 102 485 711
- US-A1- 2002 058 842
- US-A1- 2008 306 305
- US-A1- 2010 056 828
- US-A1- 2010 099 872
- US-B1- 6 376 714

## Description

### Technical Field

The present invention relates to a method for preparation of N-(2-aminoethyl) ethane-1,2-diamine.

### Background of the Invention

N-(2-aminoethyl)ethane-1,2-diamine, also named diethylenetriamine, which has molecular formula of C₄H₁₃N₃ and an abbreviation of DETA, is a saturated aliphatic amine as a hygroscopic, transparent and viscous yellow liquid having pungent ammonia-like odor and having a property of absorbing water and carbon dioxide existed in environment. It is typically used as a solvent or an organic intermediate for preparing absorbents of carbon dioxide, lubricant additives, emulsifiers, chemicals for photography, surfactants, textile finishing agents, paper reinforcers, complexones, ashless additives, metal chelating agents, heavy metal hydrometallurgy and noncyanide electroplating diffuser agent, brightening agents, ion exchange resins, polyamide resins and the like.

As a high active epoxy resin curing agent, N-(2-aminoethyl)ethane- 1,2-diamine is substantially used in an amount of 5 to 10 parts in laminated products, casting products, adhesives, coatings and the like. Therefore, it has wide applications with rapid development of epoxy resin in China.

At present, there are two main methods for preparation of polyethylene polyamine which mainly contains N-(2-aminoethyl)ethane-1,2-diamine. One method is based on dichloroethane, the other is monoethanolamine method based on ethylene oxide. The dichloroethane-based method is performed in liquid phase, which has disadvantages of serious corrosion throughout the process, difficultly in separation of the products, production of large quantities of amine-containing waste water, difficulty in post-treatment, high energy consumption and severe environmental contamination. The other method is based on ethylene oxide, in which monoethanolamine (MEOA) formed from the reaction between amine and ethylene oxide, or ethylene glycol formed from the reaction between ethylene oxide and water is reduced-aminated as a starting material. In this process, its cost is high due to critical operating conditions (high pressure and high temperature), and due to the high reaction temperature, the catalyst easily forms coke and is easily inactivated, both of the conversion rate and selectivity are low, and it needs high energy consumption for separating the products which contains complex composition.

An improved method is using ethylenediamine (EDA) as a starting material to produce DETA with presence of a catalyst which contains Ni, Cu, Co, or noble metal such as Rh, Ru, Re, Pt, Pd and the like, or the combination thereof supported on silicon dioxide, alumina or zirconium dioxide, and usually with introduction of some amount of hydrogen (for example, adding 0.1 wt% hydrogen) in order to increase catalytic activity. Both selectivity and yield are increased in this method, but there still are problems that high reaction temperature is required which facilitates coke and inactivation of the catalyst, and high cost is needed due to using EDA as a starting material.

The method of hydrogenating cyanogen group to obtain the corresponding organic amine has advantageous due to its simple process, non-pollution, energy-saving and emission-reducing. US5097072 discloses a method for preparation of polyamines, in which DETA is produced by hydrogenating iminodiacetonitrile (IDAN) in N,N-dimethylacetamide (DMAC) as the solvent with the presence of Raney cobalt catalyst at 103°C under 10 Mpa, and given a yield up to 82.7%. Additionally, US2002058842 discloses DETA is prepared by hydrogenating IDAN in an autoclave in N,N-dimethylformamide (DMF) at 100°C under 90 bar, and given a selectivity of 82% and a conversion of 100%. However, the by-product, piperazine, is difficult to be separated from DMF or DMAC since either DMF or DMAC has a similar boiling point to piperazine. In addition, amides as the solvent may perform an amino exchange reaction with amines under high pressure resulting in more unnecessary side products.

WO2008104583A1 discloses a method for preparing DETA and EDA by hydrogenating the mixture of aminoacetonitrile (AAN) and IDAN. However, unnecessary impurities are introduced since AAN is readily polymerized and decomposed at room temperature, which causes the components of the product complex and inconstant, that is adverse to subsequent separation of each kind of polyethylene polyamines; in addition, HCN from decomposed ANN may further reduce the activity of the catalyst or even inactivate the catalyst. Further, US20100099872A1 discloses process for preparing an ethylene amine mixture which comprises hydrogenating an amino nitrile mixture comprising at least two α-amino nitriles, including IDAN, in an amount of at least 5% by weight in each case in the presence of a catalyst and optionally a solvent. Furthermore, US6376714B1 relates to a process for converting dinitriles to diamines and/or aminonitriles, comprising forming a reaction mixture that comprises a dinitrile; hydrogen; a catalyst comprising a Group VIII element; and one or more modifiers selected from the group of compounds consisting of quaternary ammonium hydroxides, quaternary ammonium cyanides, quaternary ammonium fluorides, quaternary phosphonium hydroxides, and quaternary ammonium thiocyanides; wherein the reaction mixture is substantially solvent-free. Similarly, US20080306305A1 refers to process for hydrogenating oligonitriles which have at least two nitrile groups in the presence of a catalyst which, before commencement of the hydrogenation, is pretreated by contacting with a compound A which is selected from alkali metal carbonates, alkaline earth metal carbonates, ammonium carbonate, alkali metal hydrogencarbonates, alkaline earth metal hydrogencarbonates, ammonium hydrogencarbonate, alkaline earth metal oxocarbonates, alkali metal carboxylates, alkaline earth metal carboxylates, ammonium carboxylates, alkali metal dihydrogenphosphates, alkaline earth metal dihydrogenphosphates, alkali metal hydrogenphosphates, alkaline earth metal hydrogenphosphates, alkali metal phosphates, alkaline earth metal phosphates and ammonium phosphate, alkali metal acetates, alkaline earth metal acetates, ammonium acetate, alkali metal formates, alkaline earth metal formates, ammonium formate, alkali metal oxalates, alkaline earth metal oxalates and ammonium oxalate. Moreover, WO2004066703A2 discloses a process for the catalytic hydrogenation of a nitrile in the presence of an amine, a hydroxide and a freshly prepared and liquid-rinsed Raney-type catalyst, characterized in that the catalyst is contacted with at least a part of the hydroxide prior to contacting the catalyst with the amine.

### Summary of the Invention

The technical problem to be solved by the present invention is to provide a method for preparation of DETA by hydrogenating IDAN, which can prevent IDAN from decomposing, increase conversion of IDAN and increase selectivity of DETA, inhibit the formation of piperazine as the by-product and some high-boiling-point products, and thereby simplify the subsequent separation.

For solving the above technical problems, the present invention provides a method for preparation of DETA comprising steps of: preparing a mixture by dissolving IDAN in an organic solvent, adding an anion exchange resin of OH type and a stabilizing agent for IDAN, and subjecting the mixture to hydrogenation at a temperature of 50-150°C, and under a pressure of 5-25 Mpa in the presence of a hydrogenation catalyst and a first additive, to obtain DETA,
wherein the organic solvent is one or two or more selected from of C₁₋₄ alcohols, ethers, hydrocarbons and amines or amides, the weight ratio of the organic solvent and IDAN ranging from 0.2:1 to 20:1, wherein the stabilizing agent for IDAN is one or two or more selected from alkali metallic oxides or alkaline earth metallic carbonates, alumina, zinc oxide, sodium tungstate, activated carbon, silica gel, zeolite or molecular sieve, the weight ratio of the stabilizing agent for IDAN and IDAN ranging from 0.05:1 to 1:1, and wherein the first additive is a basic solution and optional liquid ammonia, the molar ratio of the optional liquid ammonia and IDAN ranging from 0:1 to 10:1, and the weight ratio of the basic solution and IDAN ranging from 0.0001:1 to 0.1:1.

In a preferable embodiment, the temperature during the hydrogenation step is 70-90°C, and the pressure is 9-14 Mpa.

The organic solvent is selected from one or two or more of C₁-C₄ alcohols, ethers, hydrocarbons and organic amines or amides, preferably selected from one or two or more of methanol, ethanol, propanol, ethylene glycol, diethylene glycol, tetrahydrofuran, oxanes, morpholine, 1,4-dioxane, 1,3-dioxane, toluene, benzene, petroleum ether, cyclohexane, dimethylamine, trimethylamine, ethylamine, phenylenediamine, aniline, cyclohexylamine and 1,2-diaminoethane, more preferably selected from methanol or ethanol. When two or more of the organic solvents are used, they can be combined in any ratio. The weight ratio of the organic solvent and IDAN ranges from 0.2:1 to 20:1, preferably from 4:1 to 9:1.

IDAN may be commercially available or industrial-grade products, or a solution comprising 1-50 wt%, preferably 6-20 wt% of IDAN (for example, as the solution may be a reactant solution).

The stabilizing agent for IDAN is selected from one or two or more of alkali metallic oxides or alkaline earth metallic carbonates, alumina, zinc oxide, sodium tungstate, activated carbon, silica gel, zeolite or molecular sieve, preferably selected from calcium carbonate, barium carbonate, molecular sieve, alumina or activated carbon.

In a preferable embodiment, the stabilizing agent for IDAN may be modified in order to obtain an improved stability. In a preferable embodiment, the stabilizing agent for IDAN may usually be modified by a method comprising steps of supporting and dispersing a second additive on a stabilizing agent for IDAN (such as activated carbon, diatomite, alumina, silicon dioxide, silica gel, zeolite, molecular sieve or the like) by a method of impregnation, coprecipitation, deposition, spray-coating or ion-exchange, drying at 80-20°C, and calcining for 4-8 hours at 400-850°C.

The second additive for the modification is selected from alkali metallic oxides or alkaline earth metallic oxides , hydroxides, nitrates, acetates, carbonates or fluorides, amines, cyanides, azides, or copper/zinc-containing compounds, preferably from potassium fluoride, zinc chloride, cesium acetate or potassium nitrate.

The suitable stabilizing agent for IDAN is preferably a weak base with strength of H₋>9, more preferably a moderate base with strength of H₋>17, in which the base strength is measured by the method of Hammett indicator.

The weight ratio of the stabilizing agent for IDAN and IDAN is ranging from 0.05:1 to 1:1, preferably from 0.1:1 to 0.5:1.

The anion exchange resin of OH type is a strongly basic anion exchange resin, which may be commercially provided or converted from other types of anion exchange resins by conventional methods in the art. Said other type of anion exchange resins may preferably be strongly basic quaternary ammonium type I anion exchange resins (for example, 201×7), weakly basic quaternary ammonium type I anion exchange resins (for example, 201×2, 201×4, 201×8), weakly basic epoxy-based anion exchange resins (for example, 331), macroporous strongly basic quaternary ammonium type I anion exchange resins (for example, D201), macroporous strongly basic quaternary ammonium type II anion exchange resins (for example, D202), macroporous weakly basic styrene-based anion exchange resins (for example, D302) or macroporous weakly basic acrylic acid-based anion exchange resins (for example, D311). All the above types of ion exchange resins are described in Chinese National Standard "Classifying, Naming and Types for Ion Exchange Resins", GB1631-79. The anion exchange resin of OH type may more preferably be macroporous strongly basic quaternary ammonium type I anion exchange resins (for example, D201), strongly basic quaternary ammonium type I anion exchange resins (for example, Amberlite IRA-400, Lewatit MP-60) or macroporous strongly basic quaternary ammonium type II anion exchange resins (for example, D202), most preferably D201, D202, Amberlite IRA-400 or Lewatit MP-60.

In a preferable embodiment, the water content of the OH type or other type of anion exchange resin is preferably in the range of 40-70%. The effective particle size of the resin is controlled in the range of 0.03-5 mm, preferably 0.3-1.25 mm, and the full exchange capacity of the resin is preferably not less than 4.0 mmol per gram of dry resin.

In a preferable embodiment, the highest tolerable temperature of the anion exchange resin of OH type is in the range of 60-140°C.

In a preferable embodiment, the weight ratio of the anion exchange resin of OH type and IDAN is ranging from 0.01:1 to 1:1, preferably from 0.1:1 to 0.4:1.

The above said hydrogenation catalyst is normal hydrogenation catalyst in the art, and is generally selected from two types of catalysts. One type is a supported catalyst, in which an active component which is at least one metal selected from Subgroup I, Group VIII, and Subgroup VII, is coated on a supporter which may be one or two or more selected from alumina, diatomite, silicon dioxide, silica gel, bauxite, silicate, alumina-containing zeolite, zirconium oxide, thorium dioxide. Generally, when two or more kinds of said active components are adopted, they can be mixed in any ratio. The content of said active metal(s) is in the range of 0.01-30 wt%, preferably 0.4-5 wt% based on the total weight of the catalyst. The other type is Raney cobalt or Raney nickel catalyst, in which the content of the active component, Ni or Co, is in the range of 40-90 wt%, and the size of the catalyst is ranging from 1 µm to 1 mm.

In a preferable embodiment, the Raney cobalt or Raney nickel catalyst is amorphous alloy containing Co or Ni, the X-ray diffraction spectrum diagram (XRD) of which shows only a diffuse peak (the peak position 2θ=45°) or no obvious peaks. The weight ratio of the whole hydrogenation catalyst and IDAN is ranging from 0.01:1 to 1:1, preferably from 0.1:1 to 0.5:1.

The first additive is an alkaline basic solution and optional liquid ammonia. The base forming the basic solution may be one or two or more selected from hydroxides or alcoholates of alkali metals or alkaline earth metals, or may be one selected from quaternary ammonium bases or alkaloids. The term "optional liquid ammonia" means the first additive may contain liquid ammonia or not. Generally, when using two or more of said bases, they can be combined in any ratio. The base preferably is potassium hydroxide, sodium hydroxide, tetramethylammonium hydroxide or calcium hydroxide.

The concentration of the basic solution is in the range of 0.01-50 wt%, preferably 1-20 wt%, and more preferably 2-5 wt%. The solvent for the basic solution may be organic solvents such as alcohols, ethers, amines, hydrocarbons and amides; or water, preferably methanol, ethanol or water.

The molar ratio of liquid ammonia and IDAN is ranging from 0:1 to 10:1, preferably from 0.5:1 to 8:1, more preferably from 1:1 to 6:1. The weight ratio of the basic solution and IDAN is ranging from 0.0001:1 to 0.1:1, preferably from 0.005:1 to 0.1:1, more preferably from 0.01:1 to 0.1:1.

Preferably, the method of the present invention is performed in a hydrogenating reactor consisting of a single autoclave or multiple autoclaves in series.

Anhydrous piperazine as the by-product is produced by the method of the present invention simultaneously.

The present invention has the following advantageous effects.
i) In the commercially provided IDAN, 0.1-0.2% of sulfates and a trace amount of cyanides are remained due to the limitation of the process for preparation of IDAN. These residuals poison the hydrogenation catalyst. After adding the anion exchange resin to the reaction system, said sulfate and cyanide ions are exchanged with hydroxide ions, which eliminates the poisoning factors for the catalyst, and improves the catalytic effect. Simultaneously, the macroporous resin such as macroporous strongly basic quaternary ammonium type I anion exchange resins(for example, D201), macroporous strongly basic quaternary ammonium type II anion exchange resins(for example, D202) may also adsorb the trace amount of polymer impurities formed during the process, and thereby protect the catalyst and prolong the service life of the catalyst,.
ii) IDAN tends to decompose and polymerize to form cyanide ions and high-boiling products respectively by heating, in which the cyanide ions may poison the catalyst, and the high-boiling products may be adsorbed on the surface of the catalyst to decrease its activity. By adding the alkaline stabilizing agent, the polymerization and decomposition of IDAN are both inhibited obviously, so that utilization rate of the starting material is increased and the service life of the catalyst is prolonged.
iii) In comparison with the traditional process of dehydration condensation of ethylenediamine for preparation of piperazine in which water is inevitably generated, the process of the present invention avoids generating water by hydrogenating reaction so as to produce anhydrous piperazine directly, thereby simplify the process for preparing anhydrous piperazine.
iv) The present process increases the conversion of the starting material and the selectivity of DETA by adding liquid ammonia and the basic solution. Particularly, the conversion of IDAN is 99% or higher, and the selectivity of DETA is above 89%. Generation of the by-product of piperazine and some other high-boiling products can be inhibited accordingly, and thus the subsequent separation is simplified.

### Detailed Description of the Invention

The present invention will be described with reference to the following examples in detail.

### Transformation of ion exchange resins

In two glass beakers were respectively added 100 g of anion exchange resins other than OH type (for example, Cl type). The resins were then impregnated several times in 5-20% (w/w) NaOH solution, and washed several times by distilled water until no chloride ions. Thus, the resins were transformed to the corresponding OH type for later use.

### Pretreatment of molecular sieve

Wash by a basic solution: The particles of molecular sieve were exchanged in 0.1-2 mol/L of an alkali metal salt solution for 3 to 6 times with a solid-to-liquid ratio of 2-20 at 40-80°C, in which the alkali metal salt may be an inorganic acid salt or an organic acid salt of the alkali metal, such as potassium nitrate, cesium acetate or the like.

Calcination: The above obtained molecular sieve was filtered, dried at the temperature of 80-120°C, then calcined at the temperature of 400-850°C, and cooled.

Nitrogenization: The cooled molecular sieve was nitrogenized in a high-temperature nitriding fumacein which nitrogen was filled, for 3 to 6 hours at the temperature of 300-500°C, and cooled in a dryer.

### Example 1:

Transformation of ion exchange resin: macroporous Strongly basic quaternary ammonium type I anion exchange resin(available from Hebei Langfang Jinda Chemical Plant, D201) was impregnated in 3.2%(w/w) NaOH solution for 3 times with 400 ml for each time (3×400 ml), then washed by distilled water until no chloride ions to obtain the corresponding OH type resin for later use.

Modification of alumina: 6 g of KF crystal (available from Yanyu (Shanghai) Chemical Reagent Co., Ltd) and 7.2 g Al₂O₃ (basic alumina, available from Qingdao Gaojing Chemical Co., Ltd, 200-300 mesh) were mixed, sirred for 10 min in 12 ml water at room temperature, dried by a rotary evaporator and activated at 120°C under vacuum for 10 hours. The resulting KF modified alumina was determined to have H. of 18.4.

In 1L stainless high-pressure reactor were added the mixture of 0.6 g of the transformed ion exchanged resin, 60 g of the KF modified alumina, 20 g of amorphous cobalt catalyst (available from Dalian Tongyong Chemical Co., LTD, RTH-6110, containing 97.5wt% of Co), 60 g IDAN, 400 ml methanol and 12.0 ml of 0.1 wt% KOH-methanol solution. H₂ was introduced into the reactor until the pressure reached 5.0 Mpa. The mixture was stirred at a speed of 300 rmp and heated to 60°C. At this time, the pressure of the reaction system was 8.0 Mpa. H₂ was then further introduced into the reactor until the pressure reached 16.0 Mpa, which was maintained for about 5 hours until H₂ was nearly not consumed. The reaction system was then cooled to room temperature, and the pressure was reduced to 2-3 MPa via the vent valve. The resulting solution, colorless clear liquid, was collected from the liquid outlet. Determined by GC (the following results were normalized after removing the solvent and additives, similarly hereinafter), the conversion of IDAN was 99.99%, the selectivity of DETA was 90.84%, and the selectivity of piperazine was 6.27%.

### Example 2:

In 1L stainless high-pressure reactor were added the mixture of 50 g of the anion exchange resin (available from Rohm and Haas Company, USA, Amberlite IRA-400, OH type), 0.3 g of the modified activated carbon (H₋=26.5, produced by impregnating No. 2 analysis filter paper (Toyo Co., LTD, Japan) with ZnCl₂ and calcining the result for 2 hours at 700°C in vacuum), 20 g of cobalt catalyst supported on diatomite (available from Süd-Chemie AG, German, T4724), 50 g IDAN, 400 ml methanol and 2.2 ml of 40% KOH aqueous solution. After the atmosphere of the reaction system was exchanged by N₂, 300 g of liquid ammonia was added into the reaction system which was heated while stirring, and then H₂ was introduced into the reactor. The reaction was performed at the temperature of 140°C and under the pressure of 24 Mpa. After the reaction was completed, the solution was collected and analyzed by GC. The conversion of IDAN was 100%, the selectivity of DETA was 94.25%, and the selectivity of piperazine was 2.54%.

### Example 3:

In 1L stainless high-pressure reactor were added the mixture of 12 g of the anion exchange resin (available from Lanxess Chemcal (Shanghai) Co., LTD, Lewatit MP-60, OH type), 0.3 g of the modified molecular sieve of Y type (H₋=17.2, produced by treating the molecular sieve powder of Na-Y type (Wenzhou Catalyst Plant) with 1.7 mol/L KNO₃ solution at a solid-to-liquid ratio of 20:1 for 6 times at 70°C, calcining for 5 hours at 850°C after drying at 120°C, and cooling in a dryer), 0.64 g Ru/alumina catalyst containing 0.3% Ru (available from Süd-Chemie AG, German, Nobel Max^{o,R}420), 60 g IDAN and 400 ml ethanol. Then, 7.6 ml of 5% KOH ethanol solution was added. The atmosphere of the reaction system was exchanged by N₂. After 34 g liquid ammonia was added into the reaction system which was heated while stirring, H₂ was introduced into the reactor. The reaction was performed at the temperature of 90°C and under the pressure of 14 Mpa. After the reaction was completed, the solution was collected and analyzed by GC. The conversion of IDAN was 100%, the selectivity of DETA was 95.52%, and the selectivity of piperazine was 2.17%.

### Example 4:

Transformation of the ion exchange resin: Macroporous strongly basic quaternary ammonium type I anion exchange resin(Cl type, available from Hebei Langfang Jinda Chemical Plant, D201) was impregnated with 18%(w/w) NaOH solution (3×100 ml), washed by distilled water until no Cl ions to obtain the corresponding OH type resin for later use.

In 1L stainless high-pressure reactor were added the mixture of 12 g of the transformed anion exchange resin, 0.3 g of the modified alumina (H₋=26.5, produced by immersing 200 ml alumina (basic alumina, available from Qingdao Gaojing Chemical Co., LTD, 200-300 mesh) in 300 ml of 20%(w/w) K₂CO₃ solution, and calcining for 4 hours at 600°C), 30 g Raney cobalt (available from Süd-Chemie AG, ACTICA®3100), 60 g IDAN and 400 ml ethanol. Then 7.6 ml of 5% KOH-ethanol solution was added. The atmosphere of the reaction system was exchanged by N₂. After 34 g of liquid ammonia was added into the reaction system which was heated while stirring, H₂ was introduced into the reactor. The reaction was performed at the temperature of 70°C and under the pressure of 9 Mpa. After the reaction was completed, the solution was collected and analyzed by GC. The conversion of IDAN was 100%, the selectivity of DETA was 96.05%, and the selectivity of piperazine was 1.48%.

### Example 5:

The anion exchange resin (available from Dandong Mingzhu Special Type Resin Co., LTD, D202) was treated according to the same process of transformation as in Example 1. In 1L stainless high-pressure reactor were added the mixture of 12 g of the transformed resin, 10 g of the cesium acetate treated molecular sieve of Y type (H₋=37, produced by impregnating the molecular sieve powder of Na-Y type (Wenzhou Catalyst Plant) with 1.2 mol/L cesium acetate solution at a solid-to-liquid ratio of 1:10 for 5 times at 65°C, calcining for 5 hours at 650°C after drying at 120°C, and cooling in a dryer), 30 g Raney cobalt (available from Süd-Chemie AG, German, ACTICA®3100), 60 g IDAN and 400 ml ethanol. Then 7.6 ml of 5% KOH ethanol solution was added. The atmosphere of the reaction system was exchanged by N₂. After 34 g of liquid ammonia was added into the reaction system which was heated while stirring, H₂ was introduced into the reactor. The reaction was performed at the temperature of 80°C and under the pressure of 12 Mpa. After the reaction was completed, the solution was collected and analyzed by GC. The conversion of IDAN was 100%, the selectivity of DETA was 97.53%, and the selectivity of piperazine was 1.03%.

### Example 6:

In 1L stainless high-pressure reactor were added the mixture of 12 g of the anion exchange resin (available from Lanxess Chemical (Shanghai) Co., LTD, Lewatit MP-60, OH type), 0.6 g calcium carbonate (H₋=15, produced by calcining calcium carbonate for 3 hours at 450°C), 0.64 g Ru/alumina catalyst containing 0.5% Ru (available from Süd-Chemie AG, German, Nobel Max®421), 60 g IDAN and 400 ml ethanol. Then 7.6 ml of 5% KOH ethanol solution was added. The atmosphere of the reaction system was exchanged by N₂. After 34 g of liquid ammonia was added into the reaction system which was heated while stirring, H₂ was introduced in to the reactor. The reaction was performed at the temperature of 100°C and under the pressure of 5 Mpa. After the reaction was completed, the solution was collected and analyzed by GC. The conversion of IDAN was 99%, the selectivity of DETA was 91.34%, and the selectivity of piperazine was 4.86%.

### Example 7:

Transformation of the ion exchange resin: The macroporous weakly basic acrylic acid based anion exchange resin (Cl type, available from Hebei Langfang Jinda Chemical Plant, D311) was impregnated in 18%(w/w) sodium hydroxide (3×100 ml), and washed by distilled water until no chloride ion to obtain the corresponding OH type resin for later use.

In 1L stainless high-pressure reactor were added the mixture of 12 g of the transformed anion exchange resin, 0.3 g of the modified zinc oxide (H₋=14.6, produced by calcining zinc oxide (analytical grade, available from Wuhan Huawei Chemical Instrument Co., LTD) for 3 hours at 750°C, then cooling in a dryer), 0.64 g Ru/alumina catalyst containing 0.3% Ru (available from Süd-Chemie AG in German, Nobel Max®420), 60 g IDAN and 400 ml ethanol. Then 7.6 ml of 5% KOH ethanol solution was added. The atmosphere of the reaction system was exchanged by N₂. After 34 g of liquid ammonia was added into the reaction system which was heated by stirring, H₂ was introduced into the reactor. The reaction was performed at the temperature of 90°C and under the pressure of 14 Mpa. After the reaction was completed, the solution was collected and analyzed by GC. The conversion of IDAN was 99%, the selectivity of DETA was 89.21%, and the selectivity of piperazine was 7.02%.

### Comparative Example 1

In 1L stainless high-pressure reactor were added the mixture of 20 g Raney nickel catalyst containing 97.8 wt% nickel, 60 g IDAN, and 400 ml methanol. H₂ was introduced into the reactor, and the reaction was performed at the temperature of 90°C and under the pressure of 12 Mpa. After the reaction was completed, the resulting brown solution was collected from the liquid outlet and analyzed. The conversion of IDAN was 87.34%, the selectivity of DETA was 40.83%, and the selectivity of piperazine was 28.57%.

### Comparative Example 2

In 1L stainless high-pressure reactor were added the mixture of 20 g Raney nickel catalyst containing 97.8 wt% nickel, 60 g IDAN and 400 ml methanol. Then 7.6 ml of 5% KOH ethanol solution was added. The atmosphere of the reaction system was exchanged by N₂. After 34 g of liquid ammonia was added into the reaction system which was heated while stirring, H₂ was introduced into the reactor. The reaction was performed at the temperature of 90°C and under the pressure of 12 Mpa. After the reaction was completed, the solution was collected and analyzed by GC. The conversion of IDAN was 92.15%, the selectivity of DETA was 70.36%, and the selectivity of piperazine was 17.01%.

## Claims

1. A method for preparation of N-(2-aminoethyl)ethane-1,2-diamine (DETA), which comprises steps of: preparing a mixture by dissolving iminodiacetonitrile (IDAN) in an organic solvent, adding an anion exchange resin of OH type and a stabilizing agent for IDAN, and subjecting the mixture to hydrogenation at a temperature of 50-150°C, and under a pressure of 5-25 Mpa in the presence of a hydrogenation catalyst and a first additive to obtain DETA,
wherein the organic solvent is one or two or more selected from of C₁₋₄ alcohols, ethers, hydrocarbons and amines or amides, the weight ratio of the organic solvent and IDAN ranging from 0.2:1 to 20:1,
wherein the stabilizing agent for IDAN is one or two or more selected from alkali metallic oxides or alkaline earth metallic carbonates, alumina, zinc oxide, sodium tungstate, activated carbon, silica gel, zeolite or molecular sieve, the weight ratio of the stabilizing agent for IDAN and IDAN ranging from 0.05:1 to 1:1, and
wherein the first additive is a basic solution and optional liquid ammonia, the molar ratio of the optional liquid ammonia and IDAN ranging from 0:1 to 10:1, and the weight ratio of the basic solution and IDAN ranging from 0.0001:1 to 0.1:1.

2. The method according to claim 1, **characterized in that**, the temperature during the hydrogenation step is 70-90°C, and the pressure is 9-14 Mpa.

3. The method according to claim 1 or 2, **characterized in that**, the stabilizing agent for IDAN is one or two or more selected from calcium carbonate, barium carbonate, molecular sieve, alumina or activated carbon; and the weight ratio of the stabilizing agent for IDAN and IDAN is ranging from 0.1:1 to 0.5:1.

4. The method according to any one of claims 1-3, **characterized in that**, the stabilizing agent for IDAN is further modified by steps of: supporting and dispersing a second additive on the stabilizing agent for IDAN by a method of impregnation, coprecipitation, deposition, spray-coating or ion-exchange, drying at 80-120°C, and calcining for 4-8 hours at 400-850°C,
wherein the second additive is selected from alkali metallic oxides or alkaline earth metallic oxides, hydroxides, nitrates, acetates, carbonates or fluorides, amines, cyanides, azides, or copper/zinc-containing compounds.

5. The method according to claim 4, **characterized in that**, the second additive is selected from KF, zinc chloride, cesium acetate or potassium nitrate.

6. The method according to any one of claims 1-5, **characterized in that**, the stabilizing agent for IDAN has a base strength of H₋>9, preferably H₋>17, determined by the method of Hammett indicator.

7. The method according to any one of claims 1-6, **characterized in that**, the weight ratio of the anion exchange resin of OH type and IDAN is ranging from 0.01:1 to 1:1, preferably from 0.1:1 to 0.4:1.

8. The method according to any one of claims 1-7, **characterized in that**, the anion exchange resin of OH type is transformed from strongly basic quaternary ammonium type I anion exchange resins, weakly basic quaternary ammonium type I anion exchange resins, weakly basic epoxy-based anion exchange resins, macroporous strongly basic quaternary ammonium type I anion exchange resins, macroporous strongly basic quaternary ammonium type II anion exchange resins, macroporous weakly basic styrene-based anion exchange resins or macroporous weakly basic acrylic acid-based anion exchange resins, preferably transformed from D201, D202, Amberlite IRA-400 or Lewatit MP-60.

9. The method according to any one of claims 1-8, **characterized in that** the molar ratio of the optional liquid ammonia and IDAN is ranging from 0.5:1 to 8:1.

10. The method according to any one of claims 1-9, **characterized in that**, the basic solution is one or two or more selected from solutions of hydroxides or alcoholates of alkali metals or alkaline earth metals, or one solution selected from solutions of quaternary ammonium bases or alkaloids; preferably selected from potassium hydroxide solution, sodium hydroxide solution, tetramethylammonium hydroxide solution or calcium hydroxide solution, wherein the concentration of the basic solution is in a range of 0.01-50 wt%, preferably 1-20 wt%, more preferably 2-5 wt%.

11. The method according to any one of claims 1-10, **characterized in that**, the hydrogenation catalyst contains an active component of at least one metal selected from Subgroup I or Group VIII or Subgroup VII coated on a supporter which is one or two or more selected from alumina, diatomite, silicon dioxide, silica gel, bauxite, silicate, alumina-containing zeolite, zirconium oxide, thorium dioxide; wherein the content of the active component is in a range of 0.01-30 wt%, preferably 0.5-5 wt%, based on the total weight of the catalyst.

12. The method according to any one of claims 1-10, **characterized in that**, the hydrogenation catalyst is Raney cobalt catalyst or Raney nickel catalyst, preferably the Raney cobalt catalyst or the Raney nickel catalyst is in a form of amorphous alloy, wherein the content of Ni or Co as the active component is in a range of 40-90 wt%, and the size of the particles of the catalyst is ranging of from 1 µm to 1 mm.

13. The method according to any one of claims 1-12, **characterized in that**, the weight ratio of the total hydrogenation catalyst and IDAN is ranging from 0.01:1 to 1:1.

14. The method according to any one of claims 1-13, **characterized in that**, the organic solvent is one or two or more selected frommethanol, ethanol, propanol, ethylene glycol, diethylene glycol, tetrahydrofuran, oxanes, morpholine, 1,4-dioxane, 1,3-dioxane, toluene, benzene, petroleum ether, cyclohexane, dimethylamine, trimethylamine, ethylamine, phenylenediamine, aniline, cyclohexylamine and ethanediamine; preferably selected from methanol or ethanol; wherein the weight ratio of the organic solvent and IDAN may be ranging from 4:1 to 9:1.

15. The method according to any one of claims 1-14, **characterized in that**, the method is performed in a hydrogenation reactor having a single autoclave or multiple autoclaves in series.

16. The method according to any one of claims 1-15, **characterized in that**, a by-product of the method is anhydrous piperazine.

## Patentansprüche

1. Verfahren für die Herstellung von N-(2-Aminoethyl)ethan-1,2-diamin (DETA), das die folgenden Schritte umfasst: Herstellen von einer Mischung durch Lösen von Iminodiacetonitril (IDAN) in einem organischen Lösungsmittel, Zugeben von einem Anionenaustauscherharz des OH-Typs und einem Stabilisierungsmittel für IDAN, und Unterziehen von der Mischung einer Hydrierung bei einer Temperatur von 50-150 °C und unter einem Druck von 5-25 MPa in Gegenwart von einem Hydrierungskatalysator und einem ersten Zusatzstoff, um DETA zu erhalten,
wobei das organische Lösungsmittel eines oder zwei oder mehrere ist, ausgewählt aus C₁₋₄-Alkoholen, Ethern, Kohlenwasserstoffen und Aminen oder Amiden, wobei das Gewichtsverhältnis von dem organischen Lösungsmittel zu IDAN von 0,2:1 bis 20:1 reicht,
wobei das Stabilisierungsmittel für IDAN eines oder zwei oder mehrere ist, ausgewählt aus Alkalimetalloxiden oder Erdalkalimetallcarbonaten, Aluminiumoxid, Zinkoxid, Natriumwolframat, Aktivkohle, Silikagel, Zeolith oder Molekularsieb, wobei das Gewichtsverhältnis von dem Stabilisierungsmittel für IDAN zu IDAN von 0,05:1 bis 1:1 reicht, und
wobei der erste Zusatzstoff eine basische Lösung und optional flüssiger Ammoniak ist, wobei das molare Verhältnis von dem optionalen flüssigen Ammoniak zu IDAN von 0:1 bis 10:1 reicht und das Gewichtsverhältnis von der basischen Lösung zu IDAN von 0,0001:1 bis 0,1:1 reicht.

2. Verfahren nach Anspruch 1, **dadurch gekennzeichnet, dass** die Temperatur während des Hydrierungsschritts 70-90 °C und der Druck 9-14 MPa beträgt.

3. Verfahren nach Anspruch 1 oder 2, **dadurch gekennzeichnet, dass** das Stabilisierungsmittel für IDAN eines oder zwei oder mehrere ist, ausgewählt aus Calciumcarbonat, Bariumcarbonat, Molekularsieb, Aluminiumoxid oder Aktivkohle; und das Gewichtsverhältnis von dem Stabilisierungsmittel für IDAN zu IDAN von 0,1:1 bis 0,5:1 reicht.

4. Verfahren nach einem der Ansprüche 1-3, **dadurch gekennzeichnet, dass** das Stabilisierungsmittel für IDAN ferner durch die folgenden Schritte modifiziert wird: Trägern und Dispergieren von einem zweiten Zusatzstoff auf dem Stabilisierungsmittel für IDAN durch ein Imprägnierungsverfahren, Copräzipitationsverfahren, Abscheidungsverfahren, Sprühbeschichtungsverfahren oder lonenaustauscherverfahren, Trocknen bei 80-120 °C und Kalzinieren bei 400-850 °C für 4-8 Stunden,
wobei der zweite Zusatzstoff aus Alkalimetalloxiden oder Erdalkalimetalloxiden, Hydroxiden, Nitraten, Acetaten, Carbonaten oder Fluoriden, Aminen, Cyaniden, Aziden oder kupfer-/zinkhaltigen Verbindungen ausgewählt wird.

5. Verfahren nach Anspruch 4, **dadurch gekennzeichnet, dass** der zweite Zusatzstoff aus KF, Zinkchlorid, Caesiumacetat und Kaliumnitrat ausgewählt wird.

6. Verfahren nach einem der Ansprüche 1-5, **dadurch gekennzeichnet, dass** das Stabilisierungsmittel für IDAN eine Basenstärke von H > 9, vorzugsweise H > 17 aufweist, bestimmt durch das Hammett-Indikator-Vefahren.

7. Verfahren nach einem der Ansprüche 1-6, **dadurch gekennzeichnet, dass** das Gewichtsverhältnis von dem Anionenaustauscherharz des OH-Typs zu IDAN von 0,01:1 bis 1:1, vorzugsweise von 0,1:1 bis 0,4:1 reicht.

8. Verfahren nach einem der Ansprüche 1-7, **dadurch gekennzeichnet, dass** Anionenaustauscherharz des OH-Typs aus stark basischen quartärem Ammonium-Typ-I-Anionenaustauscherharzen, schwach basischen quartärem Ammonium-Typ-I-Anionenaustauscherharzen, schwach basischen epoxidbasierten Anionenaustauscherharzen, makroporösen stark basischen quartärem Ammonium-Typ-I-Anionenaustauscherharzen, makroporösen stark basischen quartärem Ammonium-Typ-II-Anionenaustauscherharzen, makroporösen schwach basischen styrolbasierten Anionenaustauscherharzen oder makroporösen schwach basischen acrylsäurebasierten Anionenaustauscherharzen umgewandelt wird, vorzugsweise aus D201, D202, Amberlite IRA-400 oder Lewatit MP-60 umgewandelt wird.

9. Verfahren nach einem der Ansprüche 1-8, **dadurch gekennzeichnet, dass** das molare Verhältnis von dem optionalen flüssigen Ammoniak zu IDAN von 0,5:1 bis 8:1 reicht.

10. Verfahren nach einem der Ansprüche 1-9, **dadurch gekennzeichnet, dass** die basische Lösung eine oder zwei oder mehrere ist, ausgewählt aus Lösungen von Hydroxiden oder aus Alkoholaten von Alkalimetallen oder Erdalkalimetallen, oder eine Lösung ist, ausgewählt aus Lösungen von quartären Ammoniumbasen oder Alkaloiden; vorzugsweise ausgewählt aus Kaliumhydroxidlösung, Natriumhydroxidlösung, Tetramethylammoniumchloridhydroxidlösung oder Calciumhydroxidlösung, wobei die Konzentration von der basischen Lösung in einem Bereich von 0,01-50 Gew.-%, vorzugsweise 1-20 Gew.-%, besonders vorzugsweise 2-5 Gew.-% liegt.

11. Verfahren nach einem der Ansprüche 1-10, **dadurch gekennzeichnet, dass** der Hydrierungskatalysator eine aktive Komponente von mindestens einem Metall enthält, das aus der Nebengruppe I oder der Gruppe VIII oder der Nebengruppe VII ausgewählt und auf einen Träger geschichtet ist, der einer oder zwei oder mehrere ist, ausgewählt aus Aluminiumoxid, Diatomit, Siliciumdioxid, Silicagel, Bauxit, Silikat, aluminiumoxidhaltigem Zeolith, Zirkoniumoxid, Thoriumdioxid; wobei der Gehalt von der aktiven Komponente in einem Bereich von 0,01-30 Gew.-%, vorzugsweise 0,5-5 Gew.-% liegt, basierend auf dem Gesamtgewicht des Katalysators.

12. Verfahren nach einem der Ansprüche 1-10, **dadurch gekennzeichnet, dass** der Hydrierungskatalysator ein Raney-Kobaltkatalysator oder Raney-Nickelkatalysator ist, wobei vorzugsweise der Raney-Kobaltkatalysator oder Raney-Nickelkatalysator in Form einer amorphen Legierung vorliegt, wobei der Gehalt an Ni oder Co als die aktive Komponente in einem Bereich von 40-90 Gew.-% liegt und die Größe der Partikel von dem Katalysator von 1 µm bis 1 mm reicht.

13. Verfahren nach einem der Ansprüche 1-12, **dadurch gekennzeichnet, dass** das Gewichtsverhältnis von dem gesamten Hydrierungskatalysator zu IDAN von 0,01:1 bis 1:1 reicht.

14. Verfahren nach einem der Ansprüche 1-13, **dadurch gekennzeichnet, dass** das organische Lösungsmittel eines oder zwei oder mehrere ist, ausgewählt aus Methanol, Ethanol, Propanol, Ethylenglykol, Diethylenglykol, Tetrahydrofuran, Oxanen, Morpholin, 1,4-Dioxan, 1,3-Dioxan, Toluol, Benzol, Petrolether, Cyclohexan, Dimethylamin, Trimethylamin, Ethylamin, Phenylendiamin, Anilin, Cyclohexylamin und Ethandiamin; vorzugsweise ausgewählt aus Methanol oder Ethanol; wobei das Gewichtsverhältnis von dem organischen Lösungsmittel zu IDAN von 4:1 bis 9:1 reichen kann.

15. Verfahren nach einem der Ansprüche 1-14, **dadurch gekennzeichnet, dass** das Verfahren in einem Hydrierungsreaktor durchgeführt wird, der einen einzigen Autoklaven oder mehrere in Serie geschaltete Autoklaven aufweist.

16. Verfahren nach einem der Ansprüche 1-15, **dadurch gekennzeichnet, dass** ein Nebenprodukt des Verfahrens wasserfreies Piperazin ist.

## Revendications

1. Procédé pour la préparation de N-(2-aminoéthyl)éthane-1,2-diamine (DETA), qui comprend les étapes suivantes : préparation d'un mélange par dissolution d'iminodiacétonitrile (IDAN) dans un solvant organique, addition d'une résine échangeuse d'ions de type OH et d'un agent stabilisant pour l'IDAN, et soumission du mélange à une hydrogénation à une température de 50 à 150°C et sous une pression de 5 à 25 MPa en présence d'un catalyseur d'hydrogénation et d'un premier additif pour que soit obtenue de la DETA,
dans lequel le solvant organique est un ou deux ou plus de deux choisis parmi les alcools en C₁ à C₄, les éthers, les hydrocarbures, les amines et les amides, le rapport en poids du solvant organique à l'IDAN étant situé dans la plage allant de 0,2/1 à 20/1,
dans lequel l'agent stabilisant pour l'IDAN est un ou deux ou plus de deux choisis parmi les oxydes de métal alcalin, les carbonates de métal alcalino-terreux, l'alumine, l'oxyde de zinc, le tungstate de sodium, le charbon activé, le gel de silice, la zéolite et les tamis moléculaires, le rapport en poids de l'agent stabilisant pour l'IDAN à l'IDAN étant situé dans la plage allant de 0,05/1 à 1/1, et
dans lequel le premier additif est une solution basique et de l'ammoniac liquide optionnel, le rapport molaire de l'ammoniac liquide optionnel à l'IDAN étant situé dans la plage allant de 0/1 à 10/1, et le rapport en poids de la solution basique à l'IDAN étant situé dans la plage allant de 0,0001/1 à 0,1/1.

2. Procédé selon la revendication 1, **caractérisé en ce que** la température durant l'étape d'hydrogénation est de 70 à 90°C et la pression est de 9 à 14 MPa.

3. Procédé selon la revendication 1 ou 2, **caractérisé en ce que** l'agent stabilisant pour l'IDAN est un ou deux ou plus de deux choisis parmi le carbonate de calcium, le carbonate de baryum, les tamis moléculaires, l'alumine et le charbon activé ; et le rapport en poids de l'agent stabilisant pour l'IDAN à l'IDAN est situé dans la plage allant de 0,1/1 à 0,5/1.

4. Procédé selon l'une quelconque des revendications 1 à 3, **caractérisé en ce que** l'agent stabilisant pour l'IDAN est encore modifié par les étapes suivantes : support et dispersion d'un deuxième additif sur l'agent stabilisant pour l'IDAN par un procédé d'imprégnation, de coprécipitation, de déposition, de revêtement par pulvérisation ou d'échange d'ions, séchage à une température de 80 à 120°C, et calcination pendant 4 à 8 heures à une température de 400 à 850°C,
dans lequel le deuxième additif est choisi parmi les oxydes de métal alcalin, les oxydes de métal alcalino-terreux, les hydroxydes, les nitrates, les acétates, les carbonates, les fluorures, les amines, les cyanures, les azotures, et les composés contenant du cuivre/du zinc.

5. Procédé selon la revendication 4, **caractérisé en ce que** le deuxième additif est choisi parmi KF, le chlorure de zinc, l'acétate de césium et le nitrate de potassium.

6. Procédé selon l'une quelconque des revendications 1 à 5, **caractérisé en ce que** l'agent stabilisant pour l'IDAN a une force basique H⁻ > 9, de préférence H⁻ > 17, déterminée par le procédé de l'indicateur de Hammett.

7. Procédé selon l'une quelconque des revendications 1 à 6, **caractérisé en ce que** le rapport en poids de la résine échangeuse d'anions de type OH à l'IDAN est situé dans la plage allant de 0,01/1 à 1/1, de préférence de 0,1/1 à 0,4/1.

8. Procédé selon l'une quelconque des revendications 1 à 7, **caractérisé en ce que** la résine échangeuse d'anions de type OH est transformée à partir de résines échangeuses d'anions ammonium quaternaire de type I fortement basiques, de résines échangeuses d'anions ammonium quaternaire de type I faiblement basiques, de résines échangeuses d'anions à base d'époxy faiblement basiques, de résines échangeuses d'anions ammonium quaternaire de type I fortement basiques macroporeuses, de résines échangeuses d'anions ammonium quaternaire de type II fortement basiques macroporeuses, de résines échangeuses d'anions à base de styrène faiblement basiques macroporeuses, ou de résines échangeuses d'anions à base d'acide acrylique faiblement basiques macroporeuses, de préférence transformée à partir de D201, D202, Amberlite IRA-400 ou Lewatit MP-60.

9. Procédé selon l'une quelconque des revendications 1 à 8, **caractérisé en ce que** le rapport molaire de l'ammoniac liquide optionnel à l'IDAN est situé dans la plage allant de 0,5/1 à 8/1.

10. Procédé selon l'une quelconque des revendications 1 à 9, **caractérisé en ce que** la solution basique est une ou deux ou plus de deux choisies parmi les solutions d'hydroxydes ou d'alcoolates de métaux alcalins ou de métaux alcalino-terreux, ou une seule solution choisie parmi les solutions d'alcaloïdes ou de bases d'ammonium quaternaire ; de préférence choisie parmi une solution d'hydroxyde de potassium, une solution d'hydroxyde de sodium, une solution d'hydroxyde de tétraméthylammonium et une solution d'hydroxyde de calcium, dans lequel la concentration de la solution basique est située dans la plage allant de 0,01 à 50 % en poids, de préférence de 1 à 20 % en poids, mieux encore de 2 à 5 % en poids.

11. Procédé selon l'une quelconque des revendications 1 à 10, **caractérisé en ce que** le catalyseur d'hydrogénation comprend un composant actif d'au moins un métal choisi parmi le sous-groupe I ou le groupe VIII ou le sous-groupe VII, déposé sous forme de revêtement sur un support qui est un ou deux ou plus de deux choisis parmi l'alumine, la diatomite, le dioxyde de silicium, le gel de silice, la bauxite, le silicate, la zéolite contenant de l'alumine, l'oxyde de zirconium, le dioxyde de thorium ; dans lequel la teneur en le composant actif est située dans la plage allant de 0,01 à 30 % en poids, de préférence de 0,5 à 5 % en poids, par rapport au poids total du catalyseur.

12. Procédé selon l'une quelconque des revendications 1 à 10, **caractérisé en ce que** le catalyseur d'hydrogénation est un catalyseur au cobalt Raney ou un catalyseur au nickel Raney, de préférence le catalyseur au cobalt Raney ou le catalyseur au nickel Raney est sous la forme d'un alliage amorphe, dans lequel la teneur en Ni ou Co en tant que composant actif est située dans la plage allant de 40 à 90 % en poids, et la taille des particules du catalyseur est située dans la plage allant de 1 µm à 1 mm.

13. Procédé selon l'une quelconque des revendications 1 à 12, **caractérisé en ce que** le rapport en poids du catalyseur d'hydrogénation total à l'IDAN est situé dans la plage allant de 0,01/1 à 1/1.

14. Procédé selon l'une quelconque des revendications 1 à 13, **caractérisé en ce que** le solvant organique est un ou deux ou plus de deux choisis parmi le méthanol, l'éthanol, le propanol, l'éthylèneglycol, le diéthylèneglycol, le tétrahydrofurane, les oxanes, la morpholine, le 1,4-dioxane, le 1,3-dioxane, le toluène, le benzène, l'éther de pétrole, le cyclohexane, la diméthylamine, la triméthylamine, l'éthylamine, la phénylènediamine, l'aniline, la cyclohexylamine et l'éthanediamine ; de préférence choisis parmi le méthanol et l'éthanol ; dans lequel le rapport en poids du solvant organique à l'IDAN peut être situé dans la plage allant de 4/1 à 9/1.

15. Procédé selon l'une quelconque des revendications 1 à 14, **caractérisé en ce que** le procédé est effectué dans un réacteur d'hydrogénation ayant un seul autoclave ou de multiples autoclaves en série.

16. Procédé selon l'une quelconque des revendications 1 à 15, **caractérisé en ce qu'**un sous-produit du procédé est la pipérazine anhydre.
